# EUROPEAN PATENT APPLICATION

(11) **EP 2 548 506 A1**
(43) Date of publication of application: **23.01.2013**
(21) Application number: 11756009.4
(22) Date of filing: 10.02.2011
(51) Int. Cl.: A61B 5/11, A61B 5/0245

(54) **VITAL SIGNS DETECTOR DEVICE**

(30) Priority: 19.03.2010 JP 2010064586
(71) Applicant: Aisin Seiki Kabushiki Kaisha, Aichi 448-8650 (JP)
(72) Inventor: KOGURE, Shunsuke, Kariya-shi, Aichi 448-8650 (JP)
(74) Representative: Intès, Didier Gérard André
(86) International application number: PCT/JP2011/052836
(87) International publication number: WO 2011/114819

(57) **Abstract**

Disclosed is a biological information detection apparatus capable of restricting influence of external noise signals and suffering less detection omission and detection errors of biological signals. The biological information detection apparatus includes a plurality of detection units disposed on a support supporting a living body and configured to output signals corresponding to vibrations, a first extraction unit for extracting a signal in a first predetermined frequency range corresponding to a biological signal from the signal outputted from each detection unit, a second extraction unit for extracting a signal in a second predetermined frequency range as an external noise component from the signal outputted from each detection unit, a calculation unit for calculating an intensity value ratio between the signal in the first predetermined frequency range and the signal in the second predetermined frequency range, and a selection unit for selecting one or more from the plurality of detection units based on result of comparison between/among the intensity value ratios calculated by the calculation unit for the signals outputted from the respective detection units.

## Description

### TECHNICAL FIELD

The present invention relates to a biological information detection apparatus configured to detect biological information including biological (living body) vibrations such as breathing, heartbeat, body movement, etc.

### BACKGROUND ART

As a biological information detection apparatus for detecting living body vibrations such as breathing, heartbeat, body movement of a living body supported to a support such as a bed, a mat, a seat, etc., there is disclosed a technique including pressure sensing means for detecting pressure change generated from the living body, the pressure sensing means being disposed at pressure receiving portions for receiving the pressure from the living body and a controlling means configured to process signals from the pressure receiving means and output biological signals, the pressure receiving means being disposed at the pressure receiving portions with disposing densities thereof being rendered partially different from each other (see e.g. Patent Document 1).

Also, there is disclosed a technique including detection units distributed two-dimensionally within a detection target area of a support supporting a human body for detecting pressure variations, a filter for extracting a biological signal in a predetermined frequency range from an output from each detection unit, an intensity calculation unit for calculating the intensity value of the biological signal for each detection unit and an intensity distribution generation unit for generating an intensity distribution in which positions of respective detection units and the intensity value are correlated to each other (see e.g. Patent Document 2).

### PRIOR ART DOCUMENTS

Patent Document 1: Japanese Unexamined Patent Application Publication No. 2007-185409
Patent Document 2: Japanese Unexamined Patent Application Publication No. 2008-110032

### SUMMARY OF THE INVENTION

### OBJECT TO BE SOLVED BY INVENTION

With the biological information detection apparatus disclosed in Patent Document 1, the pressure sensing means are arranged such that the disposing densities thereof are set higher in the vicinity of the vibration source of the living body. For instance, in case pressure sensing means are disposed on a bed for detecting heartbeats of a human, these pressure sensing means will be arranged such that the disposing densities thereof may be higher at positions adjacent the heart organ of the human. However, when the vibration source of the living body has displaced from the high disposing density position of the pressure sensing means as a result of a turnover, a displacement of sleeping position or the like, detection of the desired biological signal may become difficult. Hence, there may occur detection omission or detection error for the desired biological signal.

Also, in the case of the biological information detection apparatus disclosed in Patent Document 2, biological signals in a predetermined frequency range are extracted by a filter from the outputs of detection units and based on the detected biological signals, an intensity distribution is generated. However, with this biological information detection apparatus, information regarding to what extent the output of the detection unit is affected by influence of an external noise is not obtained. Therefore, when the intensity distribution is under influence from the external noise, determination of biological signals may become difficult.

The present invention has been made in view of the above-described problems and its object is to provide a biological information detection apparatus capable of restricting influence of external noise signals and suffering less detection omission and detection errors of biological signals.

### MEANS FOR ACHIEVING OBJECT

According to a characterizing feature of a biological information detection apparatus relating to the present invention, the biological information detection apparatus comprises:
a plurality of detection units disposed on a support supporting a living body and configured to output signals corresponding to vibrations of the living body;
a first extraction unit for extracting a signal in a first predetermined frequency range corresponding to the frequency of the vibration of the living body from a biological signal outputted from each detection unit;
a second extraction unit for extracting a signal in a second predetermined frequency range set distinct from the first predetermined frequency range from the signal outputted from each detection unit;
a calculation unit for calculating an intensity value ratio between the signal in the first predetermined frequency range and the signal in the second predetermined frequency range for the signals outputted from the respective detection units; and
a selection unit for selecting one or more from the plurality of detection units based on result of comparison between/among the intensity value ratios calculated by the calculation unit for the signals outputted from the respective detection units.

With this characterizing feature, the calculation unit calculates an intensity value ratio between the signal in the first predetermined frequency range corresponding to the frequency of the vibration of the living body and the signal in the second predetermined frequency range which is set distinct from the first predetermined frequency range; then, the selection unit selects an appropriate detection unit(s) based on the intensity ratio. Therefore, the influence from external noise signal contained in the detection signal in a frequency range other than the first predetermined frequency range may be restricted. Further, even when there occurs a change in the position of the vibration source of the living body due to e.g. difference of physical properties of the living body or movement of the living body, a detection unit(s) that outputs a signal providing a high ratio between the first predetermined frequency range signal and the second predetermined frequency signal, will be selected, so that the desired biological vibration can be detected with high accuracy. Therefore, desired biological vibration can be detected and at the same time detection omission or detection error can be restricted.

Preferably, the first predetermined frequency range corresponds to at least one of the frequency of breathing of the living body, the frequency of heartbeat of the living body and the frequency of body movement of the living body.

With this characterizing feature, since the first predetermined frequency range corresponds to at least one of the frequency of breathing, the frequency of heartbeat and the frequency of body movement, desired biological vibration can be detected with high accuracy. Further, since the second predetermined frequency range is set distinct from the first predetermined frequency range, the intensity value ratio between the first predetermined frequency range signal and the second predetermined frequency range signal becomes a signal-to-noise (S/N) ratio for the desired biological vibration. Therefore, the selection unit selects a detection unit(s) having a high S/N, so that the biological vibration can be detected with high accuracy with using the selected detection unit(s).

Further, if the first predetermined frequency range is caused to correspond to the frequency of breathing of the living body, the breathing of the living body can be detected with high accuracy. Also, if the first predetermined frequency range is caused to correspond to the frequency of heartbeat of the living body, the heartbeat of the living body can be detected with high accuracy. Further, if the first predetermined frequency range is caused to correspond to the frequency of body movement of the living body, the body movement of the living body can be detected with high accuracy. And, with detection of selected one or combinations or all of these, the biological information can be detected with high accuracy.

Preferably, the support comprises a seat for a vehicle.

With this characterizing feature, if the biological information detection apparatus is provided in a vehicle seat, vibration of a living body on the vehicle seat can be detected with high accuracy.

### BRIEF DESCRIPTION OF THE DRAWINGS

[Fig. 1] is a block diagram showing the construction of a biological information detection apparatus according to the present invention,
[Fig. 2] is an explanatory view showing an example wherein pressure sensors are attached to a vehicle seat,
[Fig. 3] is a graph showing relationship between electric signals (potential values) outputted from first through fifth pressure sensors and elapsed times,
[Fig. 4] is a graph showing relationship between signals (potential values) processed by first through fifth low-pass filters and elapsed times, and
[Fig. 5] is a graph showing relationship between signals (potential values) processed by first through fifth high-pass filters and elapsed times.

### MODE OF EMBODYING THE INVENTION

Next, an embodiment of the present invention will be explained with reference to the accompanying drawings.

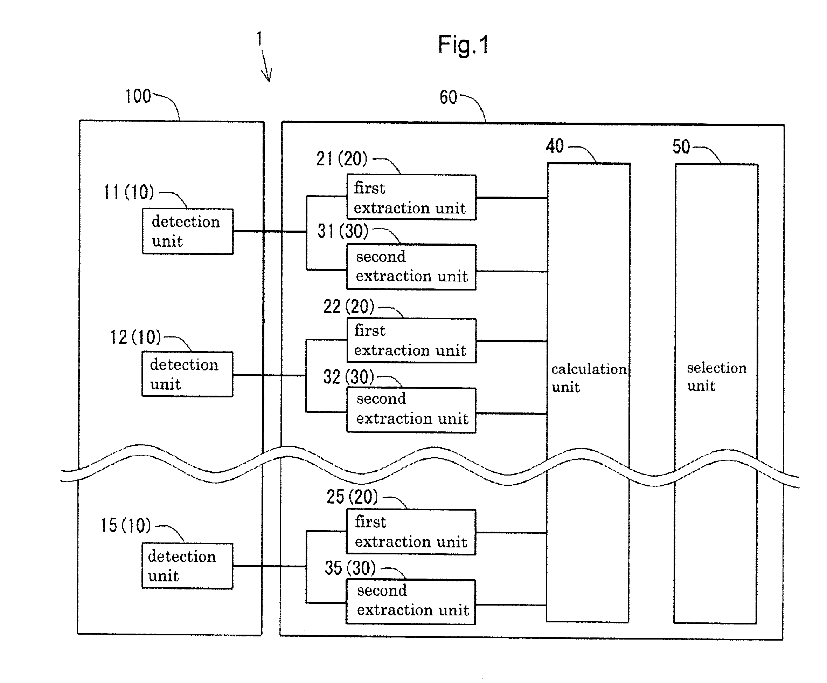

Fig. 1 a block diagram showing the construction of a biological information detection apparatus 1 according to this embodiment. A plurality of detection units 10 are disposed on a support 100 for supporting a living body and output signals corresponding to vibrations. A first extraction unit 20 extracts a signal in a first predetermined frequency range form a signal outputted from each detection unit 10. A second extraction unit 30 extracts a signal in a second predetermined frequency range form a signal outputted from each detection unit 10. A calculation unit 40 calculates a ratio between intensity values of a first predetermined frequency range signal and a second predetermined frequency range signal for the signal outputted from each detection unit 10. A selection unit 50 selects one or more units from the plurality of detection units 10 based on result of comparison between/among the intensity value ratios calculated by the calculation unit 40 for the signals outputted from the respective detection units 10. Then, the biological information detection apparatus 1 detects a signal corresponding to a vibration, with using the detection unit(s) 10 selected by the selection unit 50.

As the detection units 10, pressure sensors can be used for example. The pressure sensor outputs an electric signal corresponding to the magnitude of the pressure applied thereto. In this embodiment, the electric signals outputted from the pressure sensors include signals corresponding to change in load due to the mass of the living body present on the support, change in load due to vibrations such as breathing, heartbeat, body movement of the living body, as well as signals due to e.g. loads or vibrations not attributable to the living body. Incidentally, as the detection units 10, aside from the pressure sensors, various kinds of sensors capable of detection of change over time of the living body such as a vibration sensor can be employed also. The electric signals from the detection units 10 are outputted in bifurcation to the first extraction unit 20 and to the second extraction unit 30.

As the first extraction unit 20, e.g. a low-pass filter or a band-pass filter can be employed. In case a low-pass filter is employed as the first extraction unit 20, the cutoff frequency of this low-pass filter will be set so as to pass signals within the entire frequency range due to a biological vibration as a detection target from its expected minimal value to expected maximal value or within a portion thereof (a first predetermined frequency range). In case a band-pass filter is employed as the first extraction unit 20, the band-pass filter will be set so as to pass signals within the entire range due to a biological vibration as a detection target from its expected minimal value to expected maximal value or within a portion thereof (a first predetermined frequency range).

As the second extraction unit 30, a high-pass filter or a band-pass filter can be employed. In case a high-pass filter is employed as the second extraction unit 30, the cutoff frequency of this high-pass filter will be set so as to pass signals outside the entire frequency range due to a biological vibration as a detection target from its expected minimal value to expected maximal value or within a portion thereof (second predetermined frequency range) which range is less overlapped with the range from the expected minimal value to the expected maximal value for the signal due to a biological vibration as a detection target. In case a band-pass filter is employed as the second extraction unit 30, the band-pass frequency of this high-pass filter will be set so as to pass signals outside the entire frequency range due to a biological vibration as a detection target from its expected minimal value to expected maximal value or within a portion thereof (second predetermined frequency range) which range is less overlapped with the range from the expected minimal value to the expected maximal value for the signal due to a biological vibration as a detection target.

With the above-described setting of the frequency ranges of signals to be extracted by the first extraction unit 20 and the second extraction unit 30 (i.e. the first predetermined frequency range, the second predetermined frequency range), the first extraction unit 20 can extract mainly a signal component attributable to a biological vibration as a detection target, whereas the second extraction unit 30 can extract mainly a signal component (e.g. an external noise) other than the signal component attributable to the biological vibration as the detection target.

The calculation unit 40 can comprise an analog circuit, a digital circuit, a microprocessor, a microcomputer, etc and is configured to execute the following operations. The calculation unit 40 calculates a ratio between the intensity of the signal extracted by the first extraction unit 20 and the intensity of the signal extracted by the second extraction unit 30. More particularly, this intensity value ratio is obtained by obtaining a ratio between the maximal values selected from the signals that have been extracted by the first extraction unit 20 and the second extraction unit 30 respectively for a predetermined period which is set so as to contain at least one cycle amount of the signal due to the living body vibration as the detection target Alternatively, the intensity value ratio can be obtained also by obtaining a ratio of amplitudes of the signals that have been extracted by the first extraction unit 20 and the second extraction unit 30 respectively for the predetermined period. Further alternatively, the intensity value ratio can be obtained also by obtaining a ratio between values obtained by time-integration of respective waveforms of the signals that have been extracted by the first extraction unit 20 and the second extraction unit 30 respectively for the predetermined period. Since the signal component attributable to the biological vibration as the detection target is obtained mainly by the first extraction unit 20 and the component other than the signal component attributable to the biological vibration as the detection target is obtained mainly by the second extraction unit 30, the ratio between the intensity value of the signal extracted by the first extraction unit 20 and the intensity value of the signal extracted by the second extraction unit 30 is provided as the so-called signal-to-noise (S/N) ratio.

The selection unit 50 can comprise e.g. an analog circuit, a digital circuit, a microprocessor, a microcomputer, etc and is configured to execute the following operations. The selection unit 50 compares the ratios between the intensity values of the signals extracted by the first extraction unit 20 and the intensity values of the signals extracted by the second extraction unit 30 calculated by the calculation unit 40 for the respective detection units 10 and selects one or more detection unit(s) 10 providing higher or highest ratio value. Also, the selection unit 50 compares, for all the detection units 10, the ratios between the intensity values of the signals extracted by the first extraction unit 20 and the intensity values of the signals extracted by the second extraction unit 30 to identify of which being greater than the other and selects one or more detection unit(s) 10 providing higher or highest ratio value.

Alternatively, the selection unit 50 may be configured to select a detection unit(s) 10 that provide(s) the ratio between the intensity values of the signals extracted by the first extraction unit 20 and the intensity values of the signals extracted by the second extraction unit 30, which ratio exceeds a predetermined reference value. Further alternatively, the selection unit 50 can be configured as follows. Namely, after the unit selects one or more detection unit(s) 10 that provides the ratio between the intensity values of the signals extracted by the first extraction unit 20 and the intensity values of the signals extracted by the second extraction unit 30 which exceeds a predetermined reference signal and then effects comparison of which being greater between the intensity values of the signals extracted by the first extraction unit 20 and the intensity values of the signals extracted by the second extraction unit 30 and selects one or more detection unit(s) 10 providing large ratio values.

Then, with using the detection unit(s) 10 selected by the selection unit 50, the biological information detection apparatus 1 detects vibration due to breathing, heartbeat, body movement of the living body present on the support.

According to the instant embodiment, from the plurality of detection units 10, one or more detection unit(s) 10 that output signals having large intensity value ratio between the signal in the first predetermined frequency range and the signal in the second predetermined frequency range is/are selected. Then, with using this (or these) detection unit(s) 10, signals attributable to the living body can be detected with high accuracy. Therefore, desired biological signals can be detected and detection omission thereof can be restricted.

Incidentally, the selection of the detection unit(s) 10 by the selection unit 50 can be executed for each occasion of biological vibration detection. Instead, after selection of the detection unit(s) 10 by the selection unit 50, detection of biological vibration can be continued with using the selected detection unit(s) 10. In this case, the frequency of calculation for selection of the detection unit 10 by the selection unit 50 is reduced, so that the amount of calculation for the selection of detection unit(s) 10 can be reduced correspondingly. As a result, the detection time can be increased. Since the position of source of vibration of the living body varies in response to body movement of the living body, the configuration can be adapted for enabling detection of body movement of a living body. So that, upon detection of body movement of the living body, the selection unit 50 will execute selection of the detection unit(s) 10, and the detection of vibration attributable to the living body may be continued until next detection of body movement of the living body. In this way, in case the biological vibration detection with using the selected detection unit(s) 10 is continued for a predetermined period after the selection of the detection unit(s) 10 by the selection unit 50 and also in case the selection unit 50 executes selection of the detection unit(s) 10 in response to detection of biological vibration and the detection of vibration attributable to the living body may be continued until next detection of body movement of the living body, the number of the detection units 10 employed is reduced, which leads to power saving, yet achieves the highly accurate biological vibration detection advantageously.

Next, there will be described an example in which the biological information detection apparatus 1 according to the instant embodiment is applied to a vehicle seat 100.

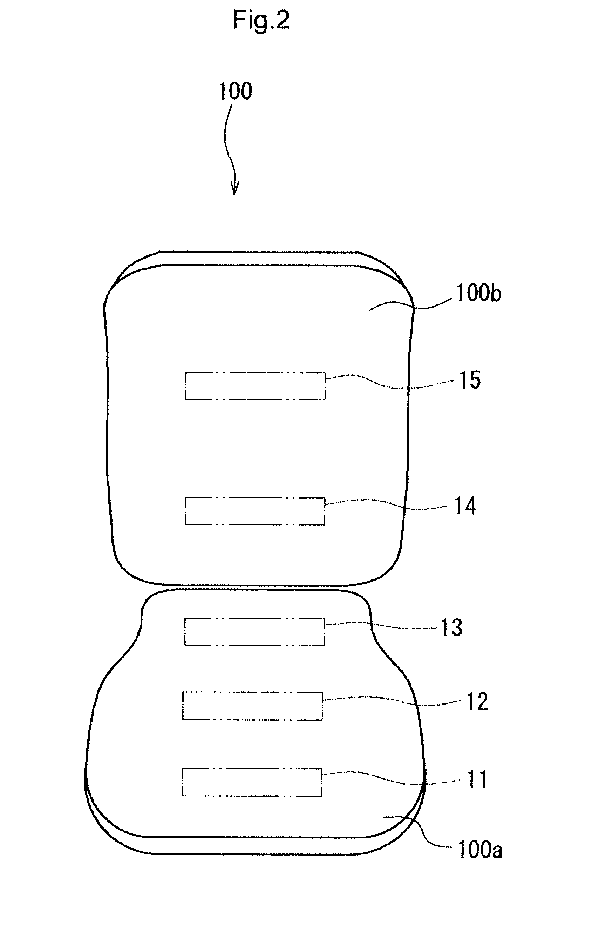

Fig. 2 is an explanatory view showing an example wherein first through fifth pressure sensors 11-15 as the detection units 10 are attached to a vehicle seat 100 as the support. As shown in Fig. 2, as the detection units 10, the first pressure sensor 11, the second pressure sensor 12 and the third pressure sensor 13 are attached to a seat portion 100a of the vehicle seat 100, whereas the fourth pressure sensor 14 and the fifth pressure sensor 15 are attached to a backrest portion 100b of the vehicle seat 100.

As shown in Fig. 1, the output of the first pressure sensor 11 is bifurcated, so that one branched output thereof is transmitted via a first low-pass filter 21 as the first extraction unit 20 to a microprocessor 60 including the calculation unit 40 and the selection unit 50 and the other branched output thereof is transmitted via a first high-pass filter 31 as the second extraction unit 30 to the microprocessor 60, respectively. Similarly, the output from each one of the second through fifth pressure sensors 12-15 is transmitted in bifurcation via second through fifth low-pass filters 22-25 and via second through fifth high-pass filters 32-35 to the microprocessor 60. Incidentally, in the instant embodiment, breathing of a living body is employed as the detection target. In consideration of the breaking normally taking place from five to twenty times per minute, the cut-off frequency of the first through fifth low-pass filters 21-25 are set to 0.33 HZ and the cut-off frequency of the first through fifth high-pass filters 31-35 are set to 0.33 HZ.

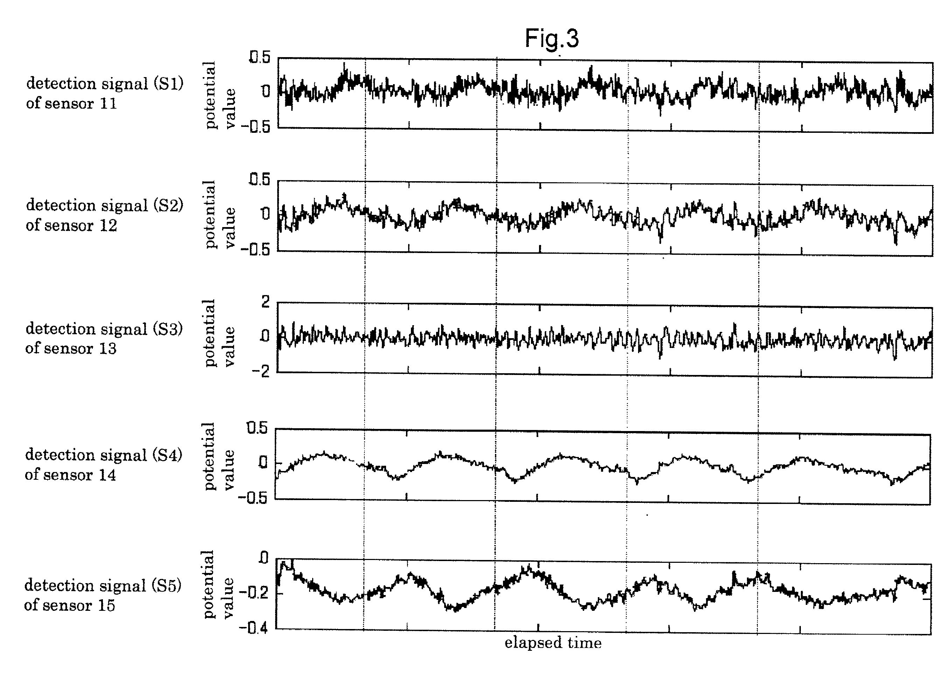

Fig. 3 is a graph showing electrical signals S1-S5 outputted from the first through fifth pressure sensors 11-15 with the horizontal axis representing lapsed time and the vertical axis representing the potential value. As shown in Fig. 3, the electric signals S1-S5 contain not only biological vibrations due to breathing, heartbeat, body movement etc. of the living body, but also non-biological vibrations not attributable to the living body.

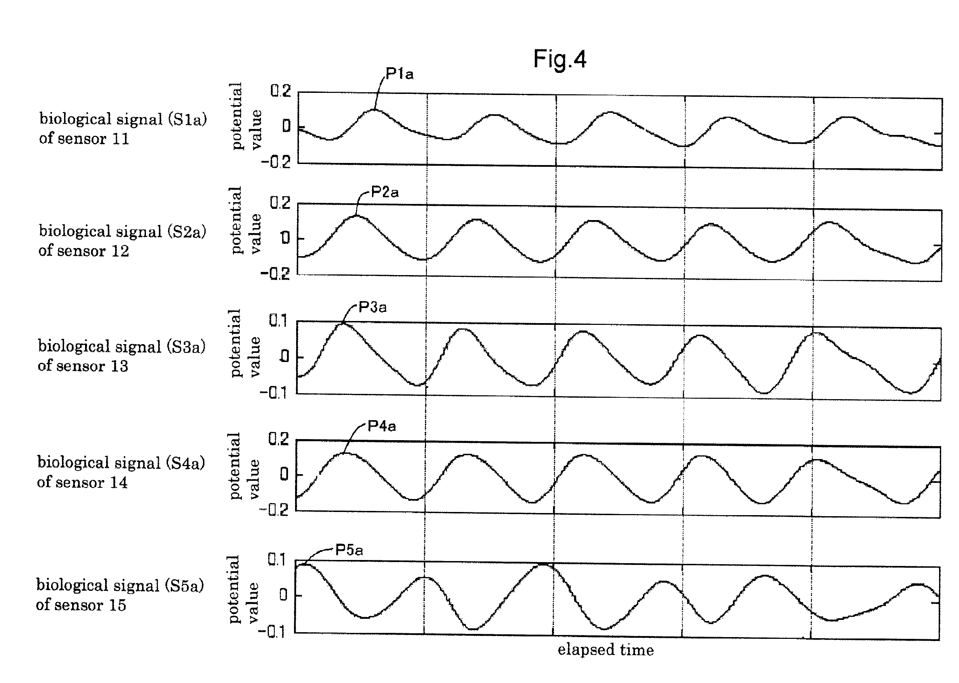

Fig. 4 is a graph showing signals S1a through S5a obtained by processing the electric signals S1-S5 outputted from the first through fifth pressure sensors 11-15 by the first through fifth low-pass filters 21-25, with the horizontal axis representing lapsed time and the vertical axis representing the potential value.

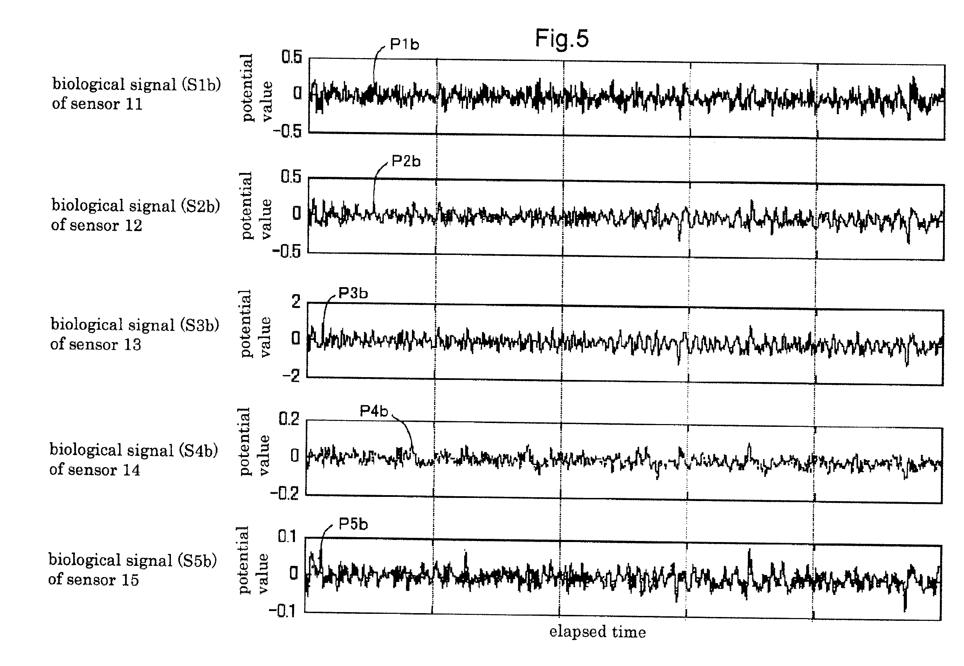

Fig. 5 is a graph showing signals S1b through S5b obtained by processing the electric signals S1-S5 outputted from the first through fifth pressure sensors 11-15 by the first through fifth high-pass filters 31-35, with the horizontal axis representing lapsed time and the vertical axis representing the potential value.

The microprocessor 60 as the calculation unit 40 inputs the signals S1a through S5a and the signals S1b through S5b. With using e.g. a peak-hold technique, the microprocessor 60 obtains the intensity values of respective peaks P1a through P5a from the signals S1a through S5a shown in Fig. 4 as maximal values within a predetermined period. Similarly, with using e.g. a peak-hold technique, the microprocessor 60 obtains the intensity values of respective peaks P1b through P5b from the signals S1b through S5b shown in Fig. 5 as maximal values within a predetermined period. Furthermore, the microprocessor 60 obtains values obtained by dividing the intensity values of the peaks P1a through P5a by the intensity values of the peaks P1 b through P5b respectively as S/N ratios of the signals obtained by the first through fifth pressure sensors 11-15.

Then, the microprocessor 60 effects, with using a desired sorting algorithm, comparison among the S/N ratios obtained by dividing the intensity values of the peaks P1a through P5a by the intensity values of the peaks P1 b through P5b respectively and selects a pressure sensor (s) having the largest S/N ratio (providing the greatest signal and the smallest noise) obtained. In the case of the example shown in Fig. 4 and Fig. 5, the value: P4a/P4b is the greatest of all, so the fourth pressure sensor 14 will be selected.

After the selection of the fourth pressure sensor 14 by the microprocessor 60, detection of vibration due to breathing of the living body is effected with using this fourth pressure sensor 14.

As described above, since a pressure sensor providing a large (or largest) S/N ratio is selected and the detection of biological vibration is effected with using this selected pressure sensor, it is possible to reduce the number of pressure sensor(s) to be employed, thus reducing the amount of calculation for the vibration detection.

Incidentally, in the instant embodiment, each one of the outputs from the first through fifth pressure sensors 11-15 is bifurcated so that the bifurcated outputs thereof are transmitted via the first through fifth low-pass filters 21-25 and the first through fifth high-pass filters 31-35, respectively. Instead of this, all of the outputs from the first through fifth pressure sensors 11-15 may be processed altogether by a signal low-pass filter or a single high-pass filter. In this case, the filtering process can be made with switching over the processing period for each one of the outputs from the first through fifth pressure sensors 11-15.

Further alternatively, the individual output from the first through fifth pressure sensors 11-15 may be subject to an analog-digital (A/D) conversion, so that a frequency analysis or a filtering process may be effected digitally.

Incidentally, in the present embodiment, the first through fifth pressure sensors 11-15 are disposed on the vehicle seat 100. However, the number of the pressure sensors to be disposed is not limited to five. It may be any other desired number greater than two. Further, in the above embodiment, the first through fifth pressure sensors 11-15 are disposed one-dimensionally along the vertical direction (the up/down direction in Fig. 2) of the vehicle seat 100. Instead, the plurality of detection units 10 may be disposed two-dimensionally, along both the vertical direction and the horizontal direction (right/left direction in Fig.2) of the vehicle seat 100. In this case, when a body movement of the living body has occurred along the horizontal direction, it is possible for the selection unit 50 to select a detection unit 10 that can detect vibration from the vibration source of the living body with high accuracy. Further, in the above embodiment, the detection units 10 are provided in both the seat portion 100a and the backrest portion 100b. Instead, the detection units 10 may be provided in only either the seat portion 100a or the backrest portion 100b. Further, the support in which the detection units 10 are to be provided is not limited to the vehicle seat 100, it may be a bed or the like, as well.

### DESCRIPTION OF REFERENCE MARKS/NUMERALS

- 1: biological information detection apparatus
- 10: detection units
- 20: first extraction unit
- 30: second extraction unit
- 40: calculation unit
- 50: selection unit
- 100: support (vehicle seat)

## Claims

1. A biological information detection apparatus comprising:
a plurality of detection units disposed on a support supporting a living body and configured to output signals corresponding to vibrations of the living body;
a first extraction unit for extracting a signal in a first predetermined frequency range corresponding to the frequency of the vibration of the living body from a biological signal outputted from each detection unit;
a second extraction unit for extracting a signal in a second predetermined frequency range set distinct from the first predetermined frequency range from the signal outputted from each detection unit;
a calculation unit for calculating an intensity value ratio between the signal in the first predetermined frequency range and the signal in the second predetermined frequency range for the signals outputted from the respective detection units; and
a selection unit for selecting one or more from the plurality of detection units based on result of comparison between/among the intensity value ratios calculated by the calculation unit for the signals outputted from the respective detection units.

2. The biological information detection apparatus according to claim 1, wherein the first predetermined frequency range corresponds to at least one of the frequency of breathing of the living body, the frequency of heartbeat of the living body and the frequency of body movement of the living body.

3. The biological information detection apparatus according to claim 1 or 2, wherein the support comprises a seat for a vehicle.
